(19) Europäisches Patentamt
European Patent Office
Office européen des brevets

(11) EP 3 572 066 B1

(12) **EUROPEAN PATENT SPECIFICATION**

(45) Date of publication and mention of the grant of the patent:
**10.07.2024 Bulletin 2024/28**

(21) Application number: **18742138.3**

(22) Date of filing: **11.01.2018**

(51) International Patent Classification (IPC):
***A61K 8/895*** (2006.01) ***A61K 8/04*** (2006.01)
***A61K 8/25*** (2006.01) ***A61K 8/39*** (2006.01)
***A61K 8/46*** (2006.01) ***A61Q 1/00*** (2006.01)
***A61Q 19/00*** (2006.01) ***C08L 83/14*** (2006.01)
***C09D 7/40*** (2018.01) ***C09D 183/07*** (2006.01)

(52) Cooperative Patent Classification (CPC):
(C-Sets available)
**A61K 8/895; A61K 8/04; A61K 8/25; A61K 8/39; A61K 8/463; A61Q 1/00; A61Q 1/10; A61Q 19/00; C08L 83/04; C09D 5/027; C09D 5/028;**
C08G 77/20; C08K 3/36 (Cont.)

(86) International application number:
**PCT/JP2018/000458**

(87) International publication number:
**WO 2018/135369 (26.07.2018 Gazette 2018/30)**

(54) **AQUEOUS SILICONE DISPERSION, COATING FILM AND COSMETIC**

WÄSSRIGE SILIKONDISPERSION, BESCHICHTUNGSFILM UND KOSMETIKUM

DISPERSION AQUEUSE DE SILICONE, FILM DE REVÊTEMENT, ET PRODUIT COSMÉTIQUE

(84) Designated Contracting States:
**DE FR GB**

(30) Priority: **20.01.2017 JP 2017008300**

(43) Date of publication of application:
**27.11.2019 Bulletin 2019/48**

(73) Proprietor: **Shin-Etsu Chemical Co., Ltd.**
**Tokyo 100-0004 (JP)**

(72) Inventor: **INOKUCHI Yoshinori**
**Annaka-shi**
**Gunma 379-0224 (JP)**

(74) Representative: **Mewburn Ellis LLP**
**Aurora Building**
**Counterslip**
**Bristol BS1 6BX (GB)**

(56) References cited:
**EP-A1- 1 361 253**
**EP-A2- 1 536 762**
**EP-A2- 2 357 024**
**WO-A1-2008/057155**
**WO-A1-2008/090458**
**WO-A1-2016/164296**
**JP-A- 2004 124 083**
**JP-A- 2004 514 755**
**JP-A- 2006 511 470**
**JP-A- 2010 174 206**
**JP-A- 2014 533 105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08L 83/04, C08K 3/36, C08K 5/04, C08L 71/02;**
C08K 3/36, C08L 83/04

**Description**

TECHNICAL FIELD

This invention relates to aqueous silicone dispersions capable of forming an elastomer film on normal temperature drying, films, and cosmetic compositions.

BACKGROUND ART

Aqueous silicone dispersions of various compositions capable of forming an elastomer film on drying are known in the art. These dispersions are used as textile treating agents, rubber coating agents, building member coating agents, paper or plastic film coating agents or additives thereto for the purpose of imparting lubricity, water repellency or releasability.

In one of the methods for forming elastomer films, condensation reaction of silicone takes place simultaneously with drying. As exemplary compositions complying with this method, USP 3,098,833 (Patent Document 1) discloses an emulsion composition comprising a hydroxylated diorganopolysiloxane, a polysiloxane having silicon-bonded hydrogen, and a curing catalyst; JP-B S38-860 (Patent Document 2) discloses an emulsion composition comprising a polydiorganosiloxane capped with hydroxyl groups at both ends of the molecular chain, a polyorganohydrogensiloxane, a polyalkyl silicate, and a tin salt of fatty acid; JP-A S53-130752 (Patent Document 3) discloses an emulsion composition comprising a polydiorganosiloxane capped with hydroxyl groups at both ends of the molecular chain, a silane having at least three hydrolysable groups, and a curing catalyst; USP 3,294,725 (Patent Document 4) discloses an emulsion composition comprising a hydroxylated diorganosiloxane, a trialkoxysilane, and colloidal silica; JP-A S54-131661 (Patent Document 5) discloses an organopolysiloxane latex composition obtained from emulsion polymerization of a cyclic organosiloxane and an organotrialkoxysilane. One common practice taken in order to form elastomer films from these compositions by drying and simultaneous condensation reaction of silicone is heating at 100 to 300°C. On drying at normal temperature, the reaction rate is slow or no reaction takes place. These compositions are unsuitable as cosmetic raw material because heating on the skin is impossible.

JP-A H07-196984 (Patent Document 6) discloses a silicone emulsion composition obtained by mixing and dispersing an emulsion of an amino-containing organopolysiloxane and a hydrolysable silane containing an epoxy group, or a silicone emulsion composition obtained by mixing and dispersing an emulsion of an epoxy-containing organopolysiloxane and a hydrolysable silane containing an amino group. Although the reaction rate of amino groups with epoxy groups is high, the amino and epoxy groups leave doubt on the safety to the skin when used in the cosmetics which are coated to the skin and kept as a cosmetic film on the skin for a long time such as make-up cosmetics and pack cosmetics.

There is a method for forming a silicone elastomer film on drying and simultaneous addition reaction of alkenylsilyl groups with hydrosilyl groups. As the composition for this method, JP-A S50-94082 (Patent Document 7) discloses an emulsion comprising a polydiorganosiloxane capped with vinyl at a molecular chain end, a polyorganohydrogenpolysiloxane, and a platinum catalyst; and JP-A S54-52160 (Patent Document 8) proposes an emulsion comprising a polydiorganosiloxane containing vinyl at a molecular chain end or side chain, a polysiloxane having silicon-bonded hydrogen, colloidal silica, and a platinum catalyst. These compositions, however, have the problem that reaction will take place or hydrogen gas generate with the lapse of time if the hydrosilyl-containing siloxane and the platinum catalyst coexist. Thus, the silicone emulsion and the platinum catalyst must be mixed prior to use. For general consumers using cosmetics, the step of mixing liquid parts prior to use is inconvenient.

JP-A S56-36546 (Patent Document 9) discloses a method of adding a platinum catalyst to an emulsion comprising a polydiorganosiloxane capped with vinyl groups at both ends of the molecular chain and a polyorganohydrogensiloxane, for thereby forming an emulsion of a crosslinked silicone elastomer, or a method of further blending colloidal silica in the silicone elastomer emulsion. Although the addition reaction of vinylsilyl groups with hydrosilyl groups is completed, this composition forms an elastomer film on drying. Regrettably, the resulting film is less extensible (or elongatable) and brittle.

Patent Document 10 discloses an emulsion of a silicone fluid having pituitous rheological properties, and the silicone fluid comprises a carrier fluid and the hydrosilylation reaction product of a SiH-containing organopolysiloxane and an alkenyl-containing organopolysiloxane. The hydrosilylation reaction product can be described as a silicone elastomer.

Patent Document 11 discloses addition curable silicone rubber compositions which cure into silicone rubber having a tack-free surface, a high modulus of resilience and bouncy rubber feel despite a low cured hardness, and being useful in the preparation of nursing bottle nipples and pacifiers. Patent Document 11 is silent about any dispersion.

Patent Document 12 discloses silicone gel forming compositions and skin adhesive silicone gels which are produced by curing the gel forming composition and provide high adhesion to skin and low peel release.

Patent Document 13 discloses anionic surfactant-containing emulsions of a fluid silicone (silicone-in-water),

but is silent about any elastomer.

Patent Document 14 discloses a cosmetic including silicone microparticles, the silicone microparticles include silicone elastomer spherical microparticles having a volume average particle diameter within a range from 0.1 to 100 μm, and a polyorganosilsesquioxane that coats a surface of the silicone elastomer spherical microparticles, and the silicone elastomer is capable of absorbing not less than 30 parts by mass of at least one oily substance selected from sebum, hydrocarbon oils and ester oils per 100 parts by mass of the silicone elastomer.

## PRIOR ART DOCUMENTS

## PATENT DOCUMENTS

Patent Document 1: USP 3,098,833
Patent Document 2: JP-B S38-860
Patent Document 3: JP-A S53-130752
Patent Document 4: USP 3,294,725
Patent Document 5: JP-A S54-131661
Patent Document 6: JP-A H07-196984
Patent Document 7: JP-A S50-94082
Patent Document 8: JP-A S54-52160
Patent Document 9: JP-A S56-36546
Patent Document 10: WO 2016164296 A
Patent Document 11: EP 1361253 A1
Patent Document 12: WO 2008057155 A1
Patent Document 13: EP 1536762 A2
Patent Document 14: EP 2357024 A2

## SUMMARY OF THE INVENTION

## TECHNICAL PROBLEM

An object of the invention, which has been made under the above-mentioned circumstances, is to provide an aqueous silicone dispersion capable of quickly forming a silicone elastomer film having elongation and strength on drying at normal temperature, a film, and a cosmetic composition.

## SOLUTION TO THE PROBLEM

Making extensive investigations to attain the above object, the inventor has found that the above object is attained by using as the silicone a silicone elastomer obtained from addition reaction of a specific alkenyl-containing organopolysiloxane with a specific hydrosilyl-containing organopolysiloxane, changing the hydrosilyl-containing organopolysiloxane so as to additionally contain a linear diorganopolysiloxane having hydrosilyl groups at both ends of the molecular chain, using an anionic surfactant as an emulsifier in a dispersion, and reducing the amount thereof.

Accordingly, the invention provides an aqueous silicone dispersion, a film, a cosmetic composition and a method of preparing the aqueous silicone dispersion, as defined in the claims.

## ADVANTAGEOUS EFFECTS OF THE INVENTION

According to the invention, there is provided an aqueous silicone dispersion capable of quickly forming a silicone elastomer film having elongation and strength on drying at normal temperature. This aqueous silicone dispersion is especially useful in cosmetics such as make-up cosmetics, pack cosmetics and eyelash cosmetics.

## DESCRIPTION OF EMBODIMENTS

Now the invention is described in detail.

COMPONENT (A)

Component (A) is a silicone elastomer which is the addition reaction product of an alkenyl-containing organopolysiloxane with a hydrosilyl-containing organopolysiloxane. The alkenyl-containing organopolysiloxane is (A-1) an alkenyl-containing organopolysiloxane having at least 2 alkenyl groups per molecule, whereas the hydrosilyl-containing organopolysiloxane includes (A-2) an organopolysiloxane having at least 3 hydrosilyl groups per molecule and (A-3) a linear diorganopolysiloxane having hydrosilyl groups at both ends of the molecular chain.

(A-1)

The alkenyl-containing organopolysiloxane having at least 2 alkenyl groups per molecule may be used alone or in combination of two or more, while it typically has the average compositional formula (1):

$$R^1{}_aR^2{}_bSiO_{(4-a-b)/2} \qquad (1)$$

wherein $R^1$ is independently a $C_1$-$C_{30}$ substituted or unsubstituted monovalent hydrocarbon group exclusive of alkenyl, $R^2$ is independently a $C_2$-$C_6$ alkenyl group, a and b are positive numbers satisfying $0 < a < 3$, $0 < b \leq 3$, and $0.1 \leq a+b \leq 3$, preferably $0 < a \leq 2.295$, $0.005 \leq b \leq 2.3$, and $0.5 \leq a+b < 2.3$.

Examples of the group $R^1$ include alkyl groups such as methyl, ethyl, propyl, butyl, pentyl, hexyl, heptyl, octyl, decyl, undecyl, dodecyl, tetradecyl, pentadecyl, hexadecyl, heptadecyl, octadecyl, nonadecyl, icosyl, heneicosyl, docosyl, tricosyl, tetracyl, and triacontyl; aryl groups such as phenyl, tolyl, and naphthyl; aralkyl groups such as benzyl and phenethyl; cycloalkyl groups such as cyclopentyl, cyclohexyl and cycloheptyl; and substituted hydrocarbon groups in which some or all of the carbon-bonded hydrogen atoms are substituted by atoms such as halogen atoms (fluorine, chlorine, bromine and iodine atoms), hydrocarbon groups substituted with a substituent such as acryloyloxy, methacryloyloxy, epoxy, glycidoxy or carboxyl, and hydrocarbon groups substituted with the aforementioned atom and substituent. It is preferred from the industrial aspect that methyl account for at least 50 mol% of the entire $R^1$ groups.

Examples of the group $R^2$ include vinyl, allyl, propenyl, butenyl, pentenyl, and hexenyl, with vinyl being preferred from the industrial aspect.

The structure of component (A-1) may be linear, cyclic or branched. The organopolysiloxane of linear structure is, for example, one having the general formula (2).

[Chem. 1]

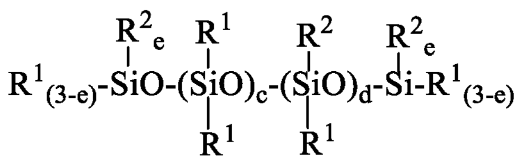

(2)

Herein $R^1$ and $R^2$ are as defined above, c is a positive number, d is 0 or a positive number, e is 0 or 1, with the proviso that d and e are numbers satisfying $d+2\times e \geq 2$.

The organopolysiloxane of branched structure is, for example, one branched from $R^1SiO_{3/2}$ unit, as represented by the general formula (3).

[Chem. 2]

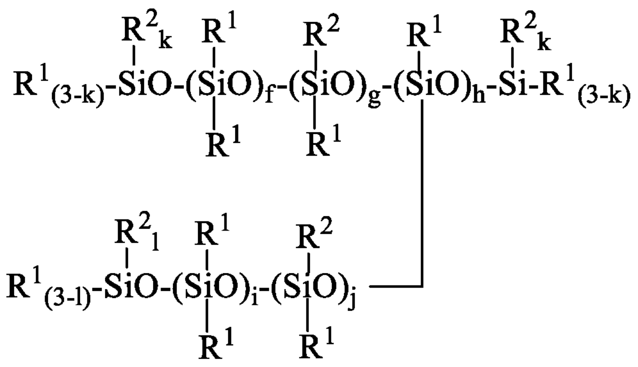

(3)

Herein $R^1$ and $R^2$ are as defined above, f is a positive number, g is 0 or a positive number, h is a positive number, i is a positive number, j is 0 or a positive number, k is 0 or 1, 1 is 0 or 1, with the proviso that g, j, k, and l are numbers satisfying $g+h\times j+2\times k+h\times l \geq 2$.

The structure branched from $SiO_{4/2}$ unit is, for example, one having the general formula (4).

[Chem. 3]

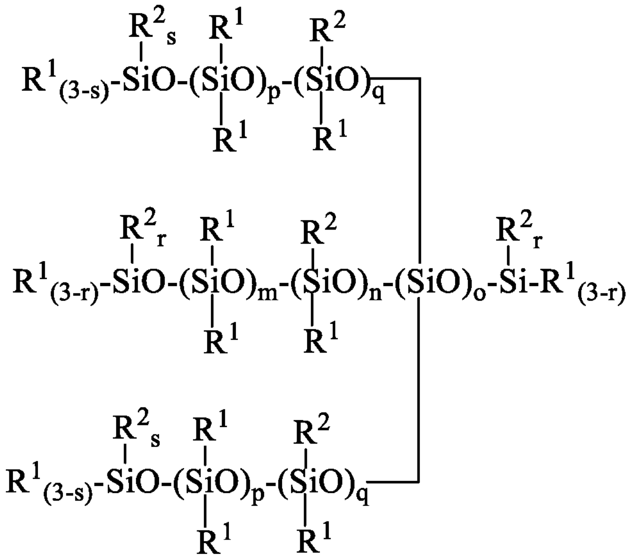

(4)

Herein $R^1$ and $R^2$ are as defined above, m is a positive number, n is 0 or a positive number, o is a positive number, p is a positive number, q is 0 or a positive number, r is 0 or 1, s is 0 or 1, with the proviso that n, q, r, and s are numbers satisfying $n+2\times o\times q+2\times r+2\times o\times s \geq 2$.

Also included are those having at least two alkenyl groups per molecule, represented by the unit formula (5).

$$[R^1{}_3SiO_{1/2}]_t[R^2(R^1)_2SiO_{1/2}]_u[SiO_{4/2}]_v \qquad (5)$$

Herein $R^1$ and $R^2$ are as defined above, t is 0 or a positive number, u is a positive number, and v is a positive number.

(A-2)

The organohydrogenpolysiloxane having at least 3 hydrosilyl groups per molecule may be used alone or in combination of two or more. For example, the organohydrogenpolysiloxane typically has the average compositional formula (6).

$R^3_wH_xSiO_{(4-w-x)/2}$

Herein $R^3$ is independently a $C_1$-$C_{30}$ substituted or unsubstituted monovalent hydrocarbon group exclusive of alkenyl, w and x are positive numbers satisfying $0 < w < 3$, $0 < x \leq 3$, and $0.1 \leq w+x \leq 3$, preferably $0 < w \leq 2.295$, $0.005 \leq x \leq 2.3$, and $0.5 \leq w+x \leq 2.3$. Examples of group $R^3$ are as exemplified above for $R^1$.

The structure of component (A-2) may be linear, cyclic or branched. The organopolysiloxane of linear structure is, for example, one having the general formula (7).

[Chem. 4]

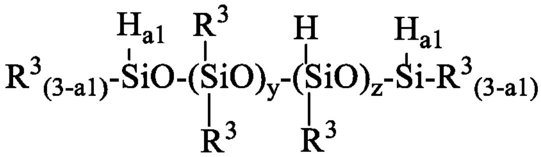

(7)

Herein $R^3$ is as defined above, y is a positive number, z is a positive number, a1 is 0 or 1, with the proviso that z and a1 are numbers satisfying $z+2\times a1 \geq 3$.

The organopolysiloxane of branched structure is, for example, one branched from $R^3SiO_{3/2}$ unit, as represented by the general formula (8).

[Chem. 5]

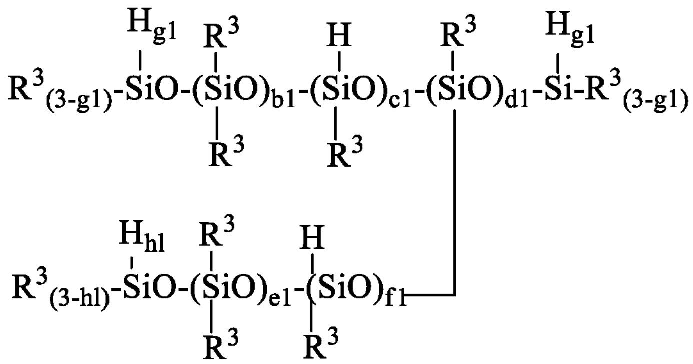

(8)

Herein $R^3$ is as defined above, b1 is a positive number, c1 is 0 or a positive number, d1 is a positive number, e1 is a positive number, f1 is 0 or a positive number, g1 is 0 or 1, h1 is 0 or 1, with the proviso that c1, f1, g1, and h1 are numbers satisfying $c1+d1\times f1+2\times g1+d1\times h1 \geq 3$.

The structure branched from $SiO_{4/2}$ unit is, for example, one having the general formula (9).

[Chem. 6]

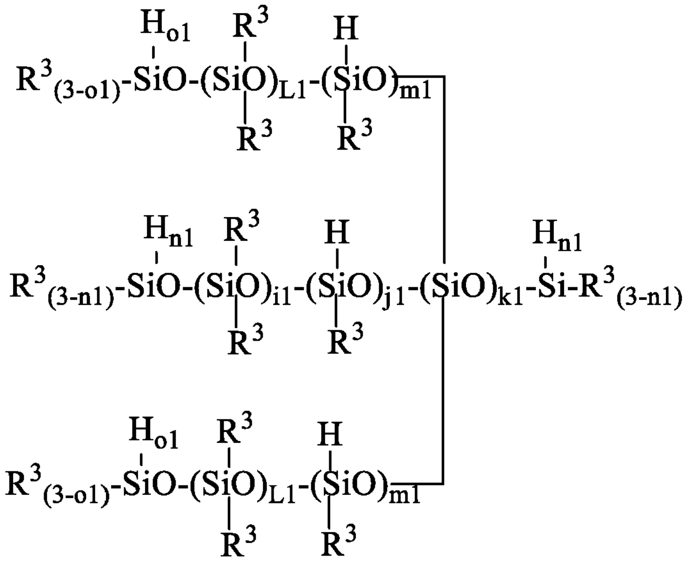

(9)

Herein $R^3$ is as defined above, i1 is a positive number, j1 is 0 or a positive number, k1 is a positive number, L1 is a positive number, m1 is 0 or a positive number, n1 is 0 or 1, o1 is 0 or 1, with the proviso that j1, m1, n1, and o1 are numbers satisfying

$$j1+2\times k1\times m1+2\times n1+2\times k1\times o1 \geq 3.$$

Also included are those having at least 3 hydrosilyl groups per molecule, represented by the unit formula (10).

$$[R^3{}_3SiO_{1/2}]_{p1}[H(R^3)_2SiO_{1/2}]_{q1}[SiO_{4/2}]_{r1} \qquad (10)$$

Herein $R^3$ is as defined above, p1 is 0 or a positive number, q1 is a positive number, and r1 is a positive number.

(A-3)

The linear diorganohydrogenpolysiloxane having hydrosilyl groups at both ends of the molecular chain may be used alone or in combination of two or more. Exemplary is a polysiloxane having 2 hydrosilyl groups per molecule, specifically represented by the general formula (11).

[Chem. 7]

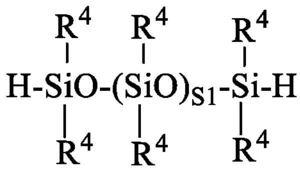

(11)

Herein $R^4$ is independently a $C_1$-$C_{30}$ substituted or unsubstituted monovalent hydrocarbon group exclusive of alkenyl, and s1 is a positive number of 5 to 1,000, preferably a positive number of 10 to 500.

Examples of group $R^4$ are as exemplified above for $R^1$.

The alkenyl-containing organopolysiloxane as component (A-1) and the organohydrogenpolysiloxane having at least 3 hydrosilyl groups per molecule as component (A-2) are not particularly limited in viscosity, and may contain a solid state compound. The linear diorganohydrogenpolysiloxane having hydrosilyl groups at both ends of the molecular

chain as component (A-3) should preferably have a kinematic viscosity at 25°C of up to 10,000 $mm^2/s$, more preferably up to 1,000 $mm^2/s$. Although the lower limit of kinematic viscosity is not critical, the viscosity may be at least 5 $mm^2/s$. A fluid obtained by mixing and dissolving components (A-1), (A-2) and (A-3) should preferably have a kinematic viscosity at 25°C of up to 10,000 $mm^2/s$, more preferably up to 1,000 $mm^2/s$. Although the lower limit of kinematic viscosity is not critical, the viscosity may be at least 5 $mm^2/s$. If the kinematic viscosity exceeds 10,000 $mm^2/s$, it may become difficult to reduce the particle size in the preparation method to be described later. It is noted that the kinematic viscosity is measured at 25°C by an Ostwald viscometer.

The alkenyl-containing organopolysiloxane as component (A-1), the organohydrogenpolysiloxane having at least 3 hydrosilyl groups per molecule as component (A-2), and the linear diorganohydrogenpolysiloxane having hydrosilyl groups at both ends of the molecular chain as component (A-3) are preferably combined in such a weight ratio that 0.5 to 2.0 moles, especially 0.8 to 1.5 moles of total hydrosilyl groups in components (A-2) and (A-3) are available per mole of alkenyl groups in component (A-1).

The organohydrogenpolysiloxane having at least 3 hydrosilyl groups per molecule as component (A-2) and the linear diorganohydrogenpolysiloxane having hydrosilyl groups at both ends of the molecular chain as component (A-3) are preferably combined such that the weight ratio of component (A-2) to component (A-3), i.e. (A-2):(A-3) may range from 5:95 to 90:10, more preferably from 10:90 to 80:20, for the reason that if the ratio of component (A-3) is too low, the resulting film may be less extensible and brittle, and if the ratio of component (A-3) is too high, no film may form, but a gel or liquid may form.

## Platinum group metal base catalyst

While the silicone elastomer as component (A) is the addition reaction product of an alkenyl-containing organopolysiloxane with a hydrosilyl-containing organohydrogenpolysiloxane, any well-known platinum group metal base catalyst may be used in the addition reaction of alkenyl groups with hydrosilyl groups. Exemplary catalysts include elemental platinum group metals such as platinum (inclusive of platinum black), rhodium and palladium; platinum chloride, chloroplatinic acid and chloroplatinic acid salts such as $H_2PtCl_4$-k'$H_2O$, $H_2PtCl_6$·k'$H_2O$, $NaHPtCl_6$·k'$H_2O$, $KHPtCl_6$·k'$H_2O$, $Na_2PtCl_6$·k'$H_2O$, $K_2PtCl_4$·k'$H_2O$, $PtCl_4$·k'$H_2O$, $PtCl_2$ and $Na_2HPtCl_4$-k'$H_2O$ wherein k' is an integer of 0 to 6, preferably 0 or 6; alcohol-modified chloroplatinic acid (see USP 3,220,972); complexes of platinum chloride or chloroplatinic acid with olefins (see USP 3,159,601, 3,159,662 and 3,775,452); complexes of chloroplatinic acid with vinyl-containing siloxanes, and complexes of platinum with vinyl-containing siloxanes; platinum group metals such as platinum black and palladium on carriers such as alumina, silica and carbon; rhodium-olefin complexes; and chlorotris(triphenylphosphine)rhodium (known as Wilkinson catalyst).

The amount of the platinum group metal base catalyst used may be an effective amount for promoting addition reaction. For example, a platinum-containing catalyst is used in such an amount as to give about 0.1 to 100 ppm (by weight), preferably about 0.5 to 50 ppm, more preferably about 1 to 30 ppm of platinum, based on the total weight of components (A-1), (A-2) and (A-3).

The silicone elastomer as component (A) may contain a silicone oil, silicone resin, organosilane, inorganic powder, organic powder, antioxidant or the like.

Since the silicone elastomer as component (A) is the addition reaction product of a specific alkenyl-containing organopolysiloxane (A-1) with specific hydrosilyl-containing organopolysiloxanes (A-2) and (A-3), the structure of the silicone elastomer is determined in a complex manner depending on the identity and ratio of components (A-1) to (A-3) used.

Since component (A) is the addition reaction product of an alkenyl-containing organopolysiloxane with hydrosilyl-containing organohydrogenpolysiloxanes, component (A) takes the form of particles dispersed in water. The particles preferably have a volume average particle size of up to 10 μm, more preferably up to 1 μm. The lower limit of particle size is not critical, with a particle size of at least 0.1 μm being acceptable. If the volume average particle size is more than 10 μm, the resulting film becomes less extensible and brittle. As used herein, the volume average particle size is measured by the laser diffraction/scattering type particle size measuring method (or system).

## COMPONENT (B)

In the practice of the invention, the anionic surfactant as component (B) functions not only as a dispersant for the silicone elastomer as component (A) in the aqueous silicone dispersion, but also as an emulsifier in emulsifying the alkenyl-containing organopolysiloxane and hydrosilyl-containing organohydrogenpolysiloxanes as reactants for component (A). The anionic surfactant may be used alone or in a combination of two or more.

Examples of the anionic surfactant include alkyl sulfate salts such as sodium laurylsulfate, polyoxyethylene alkyl ether sulfate salts, polyoxyethylene alkyl phenyl ether sulfate salts, sulfate salts of fatty acid alkylolamides, alkyl benzene sulfonate salts, polyoxyethylene alkyl phenyl ether sulfonate salts, α-olefin sulfonate salts, α-sulfofatty acid

ester salts, alkyl naphthalene sulfonic acids, alkyl diphenyl ether disulfonic acid salts, alkane sulfonic acid salts, N-acyltaurine acid salts, dialkyl sulfosuccinic acid salts, monoalkyl sulfosuccinic acid salts, polyoxyethylene alkyl ether sulfosuccinic acid salts, fatty acid salts, polyoxyethylene alkyl ether carboxylic acid salts, N-acylamino acid salts, monoalkylphosphate salts, dialkylphosphate salts, and polyoxyethylene alkyl ether phosphate salts. The alkyl sulfate salts are preferred from the standpoints of elongation and tensile strength of an elastomer film.

The amount of component (B) blended is 0.1 to 5 parts by weight, preferably 0.5 to 2 parts by weight per 100 parts by weight of the silicone elastomer as component (A). If the amount of component (B) is more than 5 parts by weight, the resulting film becomes non-extensible and brittle. If the amount of component (B) is less than 0.1 part by weight, the emulsification of reactants for component (A) fails or results in a larger particle size.

## COMPONENT (C)

In the practice of the invention, the nonionic surfactant as component (C) functions not only as a dispersant for the silicone elastomer as component (A) in the aqueous silicone dispersion, but also as an emulsifier in emulsifying the alkenyl-containing organopolysiloxane and hydrosilyl-containing organopolysiloxanes as reactants for component (A) and a dispersant for the platinum group metal base catalyst.

Examples of the nonionic surfactant include polyoxyethylene alkyl ethers, polyoxyethylene polyoxypropylene alkyl ethers, polyoxyethylene alkyl phenyl ethers, polyethylene glycol fatty acid esters, polyoxyethylene sorbitan fatty acid esters, polyoxyethylene sorbitol fatty acid esters, polyoxyethylene glycerol fatty acid esters, polyglycerol fatty acid esters, propylene glycol fatty acid esters, polyoxyethylene castor oil, polyoxyethylene hydrogenated castor oil, polyoxyethylene hydrogenated castor oil fatty acid esters, polyoxyethylene alkyl amines, polyoxyethylene fatty acid amides, polyoxyethylene-modified organopolysiloxanes, and polyoxyethylene polyoxypropylene-modified organopolysiloxanes. The nonionic surfactant may be used alone or in a combination of two or more. When two or more surfactants are used in combination, a polyether-free nonionic surfactant such as sorbitan fatty acid ester or glycerol fatty acid ester may be used in combination.

In the practice of the invention, the nonionic surfactant is optional. Since the nonionic surfactant acts to reduce the film-forming ability, a smaller amount is preferable. The amount of component (C) blended is 0 to 2 parts by weight, preferably 0 to 1 part by weight, more preferably 0 to 0.5 part by weight per 100 parts by weight of the silicone elastomer as component (A).

## COMPONENT (D)

Component (D) is colloidal silica which functions to improve the elongation and tensile strength of a silicone elastomer film. The colloidal silica is silica of nano-size, preferably having a particle size of 10 to 300 nm, more preferably 10 to 50 nm. Notably, the particle size is measured from observation under a transmission electron microscope.

In the practice of the invention, the colloidal silica used is of water dispersion type or so-called silica sol. The concentration of colloidal silica in a water dispersion is, for example, 10 to 60% by weight, though not particularly limited. Although the pH is not particularly limited, a pH value of 4 to 10 is preferred in consideration of application to the skin.

In the practice of the invention, component (D) is optional. The amount of component (D) blended is 0 to 35 parts by weight, preferably 0 to 25 parts by weight per 100 parts by weight of the silicone elastomer as component (A). If the amount of component (D) is more than 35 parts by weight, the resulting film becomes non-extensible and brittle. When component (D) is blended, its amount may be at least 5% by weight relative to 100 parts by weight of the silicone elastomer as component (A).

## COMPONENT (E)

Component (E) is water which is a dispersing medium for the silicone elastomer as component (A) and the colloidal silica as component (D). The amount of component (E) blended is 15 to 200 parts by weight, preferably 25 to 150 parts by weight per 100 parts by weight of components (A) and (D) combined. If the amount of component (E) is more than 200 parts by weight, the drying rate is so slow as to take a time for film formation. If the amount of component (E) is less than 15 parts by weight, the aqueous silicone dispersion has a high viscosity so that the dispersion becomes difficult to prepare or to handle.

## OTHER COMPONENTS

The aqueous silicone dispersion of the invention may contain a water-soluble polymer for the purpose of improving the dispersibility of component (A). The water-soluble polymer used herein is not particularly limited and encompasses nonionic water-soluble polymers, anionic water-soluble polymers, cationic water-soluble polymers, and

ampholytic water-soluble polymers.

Exemplary nonionic water-soluble polymers include copolymers of vinyl alcohol with vinyl acetate, acrylamide polymers, vinyl pyrrolidone polymers, copolymers of vinyl pyrrolidone with vinyl acetate, polyethylene glycol, isopropylacrylamide polymers, methyl vinyl ether polymers, starch, methyl cellulose, hydroxyethyl cellulose, hydroxypropyl cellulose, guar gum, and xanthane gum.

Exemplary anionic water-soluble polymers include sodium acrylate polymers, copolymers of sodium acrylate with sodium maleate, copolymers of sodium acrylate with acrylamide, sodium styrene sulfonate polymers, copolymers of sodium polyisoprene sulfonate with styrene, sodium naphthalene sulfonate polymers, carboxymethyl starch, phosphate-modified starch, carboxymethyl cellulose, sodium alginate, gum arabic, carrageenan, sodium chondroitin sulfate, and sodium hyaluronate.

Exemplary cationic water-soluble polymers include dimethyldiallylammonium chloride polymers, vinyl imidazoline polymers, methylvinylimidazolium chloride polymers, ethyl acrylate trimethylammonium chloride polymers, ethyl methacrylate trimethylammonium chloride polymers, acrylamidopropyltrimethylammonium chloride polymers, methacrylamidopropyltrimethylammonium chloride polymers, epichlorohydrin/dimethylamine polymers, ethylene imine polymers, quaternized ethylene imine polymers, allylamine hydrochloride polymers, polylysine, cation starch, cationic cellulose, chitosan, and derivatives thereof having copolymerized a monomer having a nonionic or anionic group.

Exemplary ampholytic water-soluble polymers include copolymers of ethyl acrylate trimethylammonium chloride with acrylic acid and acrylamide, copolymers of ethyl methacrylate trimethylammonium chloride with acrylic acid and acrylamide, and Hoffmann degradation products of acrylamide polymers.

The aqueous silicone dispersion of the invention may contain an antibacterial preservative or antibacterial agent. Suitable antibacterial preservatives include alkyl para-hydroxybenzoates, benzoic acid, sodium benzoate, sorbic acid, potassium sorbate, and phenoxyethanol. Suitable antibacterial agents include benzoic acid, salicylic acid, phenol, sorbic acid, alkyl para-hydroxybenzoates, p-chloro-m-cresol, hexachlorophene, benzalkonium chloride, chlorohexidine chloride, trichlorocarbanilide, photosensitizers, and phenoxyethanol.

## PREPARATION METHOD

The aqueous silicone dispersion of the invention may be prepared by emulsifying an alkeny-containing organopolysiloxane (A-1) and hydrosilyl-containing organohydrogenpolysiloxanes (A-2) and (A-3) as reactants for component (A) in water as component (E) with the aid of an anionic surfactant as component (B), adding a platinum group metal base catalyst to the emulsion and effecting addition reaction. In the emulsifying step, a nonionic surfactant as component (C) may be added.

Emulsification may be performed on a conventional emulsifier/disperser. Examples of the emulsifier/disperser include a high speed rotation centrifugal radiation type agitator such as homo-disper, a high speed rotation shear type agitator such as homo-mixer, a high pressure injection type emulsifier/disperser such as pressure homogenizer, colloidal mill, and ultrasonic emulsifier.

In an embodiment wherein the silicone elastomer as component (A) contains a silicone oil, silicone resin, organosilane, inorganic powder, organic powder and/or antioxidant, they may be previously mixed with the reactants for component (A).

While the platinum group metal base catalyst may be added after the emulsifying step as mentioned above, it is also acceptable to previously dissolve the catalyst in the reactants for component (A). Where the platinum group metal base catalyst is added after the emulsifying step, it may be dissolved in a solvent prior to addition. In case the platinum group metal base catalyst is poorly dispersible in water, it may be dissolved in the nonionic surfactant as component (C) prior to addition. Where the platinum group metal base catalyst is previously dissolved in the reactants for component (A), it is recommended to cool the solution at a low temperature of 5°C or below in order to restrain addition reaction until the completion of the emulsifying step. The addition reaction may be performed at normal temperature, for example, 20 to 25°C. If the reaction does not complete, the reaction may be performed by heating below 100°C. The agitation time for reaction is typically 1 to 24 hours through not particularly limited.

Where colloidal silica as component (D) is blended, a colloidal silica dispersion in water as dispersing medium is used as mentioned above. Preferably colloidal silica is added after the emulsifying step or after the addition reaction step.

## AQUEOUS SILICONE DISPERSION

The aqueous silicone dispersion of the invention has dispersed therein (A) a silicone elastomer which is the addition reaction product of (A-1) an alkenyl-containing organopolysiloxane having at least 2 alkenyl groups per molecule, (A-2) an organohydrogenpolysiloxane having at least 3 hydrosilyl groups per molecule, and (A-3) a linear diorganohydrogenpolysiloxane having hydrosilyl groups at both ends of the molecular chain. Although the pH of the dispersion is not particularly limited, the dispersion is preferably set at pH 4 to 10 because it is applied to the skin. As used herein,

the term "aqueous" means that a dispersion is readily diluted with water.

ELASTOMER FILM

The aqueous silicone dispersion of the invention forms an elastomer film on drying at normal temperature. The elastomer film may be tacky, but not gel. An appropriate drying temperature is selected in the range of 1 to 250°C, while a film can be formed even at room temperature, typically 25°C. The drying time is preferably several seconds to 1 week.

The rubber hardness, elongation at break and tensile strength at break of the elastomer film are defined as follows. If rubber hardness is too low, elongation is too low, or tensile strength is too low, it is believed that when the aqueous silicone dispersion is used as a pack cosmetic, it is difficult to peel a film form of the dispersion from the skin; or when the aqueous silicone dispersion is used as an eyelash cosmetic, the dispersion is readily exfoliated by rubbing.

Method for preparation of elastomer sheet

An elastomer sheet is prepared by casting the aqueous silicone dispersion into a polypropylene tray in such an amount as to give a thickness of about 1 mm after drying, and drying at 25°C for 48 hours.

Rubber hardness

The elastomer sheet prepared by the above method is measured for rubber hardness by Type A Durometer tester according to the method of JIS K 6251. Where the Durometer Type A scale rubber hardness is less than 10, rubber hardness is measured by an Asker C tester according to the testing method of the Society of Rubber Industry, Japan Standard (SRIS). The elastomer sheet preferably has an Asker C tester rubber hardness of at least 5, more preferably at least 30. Although the upper limit of rubber hardness is not critical, the Durometer Type A scale rubber hardness may be up to 60.

Elongation at break and tensile strength at break

A dumbbell shaped #3 specimen of the elastomer sheet preferably has an elongation at break of at least 20%, more preferably at least 50% as measured by the testing method of JIS K6251. The upper limit of elongation may be up to 1,000% although the upper limit is not critical. The elastomer sheet preferably has a tensile strength at break of at least 0.05 MPa, more preferably at least 0.10 MPa as measured by the testing method of JIS K6251. The upper limit of tensile strength may be up to 5.0 MPa although the upper limit is not critical.

APPLICATIONS

Since the aqueous silicone dispersion of the invention can quickly form a film of silicone elastomer having elongation and strength on drying at normal temperature, and eliminates any concern about safety to the skin because of the absence of amino and epoxy groups, the dispersion may be blended in make-up cosmetics such as foundations (inclusive of all solid and liquid forms), shadow, lipstick, lip cream, cheek, eye brow, and eye line, pack cosmetics, and eyelash cosmetics such as mascara. The dispersion is also useful as an anti-transfer agent for make-up cosmetics, a film forming agent for film-forming pack cosmetics, and a lubricating or volume-imparting agent for eyelash cosmetics. Although the amount of the aqueous silicone dispersion in a cosmetic composition is not particularly limited, the dispersion is blended in an amount of 10 to 95% by weight. In the embodiment wherein the dispersion is used in a cosmetic composition, examples of the object on which a film is formed include the skin, hair, nail and eyelash. Suitable other applications include water-repellents, water-proof agents, and parting agents for paper, water-repellent and hand-modifying agents for textile, water-repellent and water-proof agents for concrete, mortar and wood, and binders for coating agents containing inorganic particles such as titanium oxide particles.

EXAMPLES

Examples and Comparative Examples are given below for further illustrating the invention. In Examples, the kinematic viscosity is a value measured at 25°C by an Ostwald viscometer. All percent (%) representative of concentration and content are by weight.

The method for preparing elastomer sheet, and the methods for measuring rubber hardness, elongation at break, and tensile strength at break are shown below.

Method for preparing elastomer sheet

An elastomer sheet was prepared by casting an aqueous silicone dispersion into a polypropylene tray in such an amount as to give a thickness of about 1 mm after drying, and drying at 25°C for 48 hours.

Method for measuring rubber hardness

The elastomer sheet was stripped from the tray and measured for rubber hardness by a Type A Durometer tester according to the method of JIS K 6251. Where the Durometer Type A scale rubber hardness was less than 10, rubber hardness was measured by an Asker C tester according to the testing method of the Society of Rubber Industry, Japan Standard (SRIS).

Method for measuring elongation at break and tensile strength at break

A dumbbell shaped #3 specimen of the elastomer sheet was measured for elongation at break and tensile strength at break by the testing method of JIS K6251.

Example 1

A glass beaker of volume 1 L was charged with 271 g of dimethylpolysiloxane containing vinyl at both ends of the molecular chain and having a kinematic viscosity of 130 $mm^2/s$ and a vinyl content of 0.035 mol/100 g, as represented by the formula (A-1') shown below, 137 g of hydrosilyl-containing methylhydrogenpolysiloxane having a kinematic viscosity of 430 $mm^2/s$ and a hydrosilyl content of 0.040 mol/100 g, as represented by the formula (A-2') shown below, and 92 g of linear dimethylhydrogenpolysiloxane containing the hydrosilyl at the two ends of the molecular chain and having a kinematic viscosity of 35 $mm^2/s$ and a hydrosilyl content of 0.066 mol/100 g, as represented by the formula (A-3') shown below, which were stirred at 2,000 rpm for dissolution by means of a homo-mixer. The total number of hydrosilyl groups on the hydrosilyl-containing methylhydrogenpolysiloxane of formula (A-2') and the linear dimethylhydrogenpolysiloxane containing hydrosilyl at both ends of the molecular chain of formula (A-3') is 1.22 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3') = 60:40.

[Chem. 8]

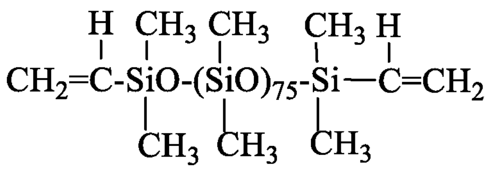

(A-1')

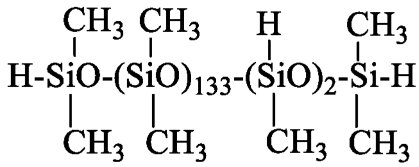

(A-2')

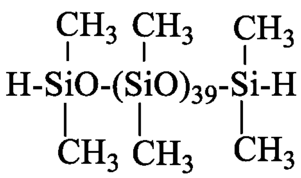

(A-3')

Next, 5 g (corresponding to 1.0 part by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 353 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 8 g of phenoxyethanol as an antibacterial agent was added to the emulsion and agitation was continued at the temperature for 10 minutes, yielding an aqueous silicone dispersion. The content of water in the aqueous silicone dispersion was 96.6 parts by weight per 100 parts by weight of overall component (A).

On measurement by a laser diffraction/scattering method particle size distribution measuring system LA-960 (Horiba, Ltd.), the silicone elastomer in the aqueous silicone dispersion had a volume average particle size of 900 nm.

The aqueous silicone dispersion was dried by the above-mentioned method, obtaining a tacky sheet. The sheet had an Asker C rubber hardness of 8, an elongation at break of 65%, and a tensile strength at break of 0.08 MPa.

About 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

Example 2

To the aqueous silicone dispersion obtained as in Example 1, was added 53 g (corresponding to 5.3 parts by weight of colloidal silica per 100 parts by weight of the silicone elastomer) of a colloidal silica water dispersion having a concentration of 40% (trade name COSMO S-40, by JGC Catalysts and Chemicals Ltd (JGC C&C)). This was agitated for 10 minutes, yielding an aqueous silicone dispersion. The content of water in the aqueous silicone dispersion was 96.9 parts by weight per 100 parts by weight of component (A) and colloidal silica combined.

The aqueous silicone dispersion was dried by the above-mentioned method, obtaining a tacky sheet. The sheet had an Asker C rubber hardness of 26, an elongation at break of 130%, and a tensile strength at break of 0.15 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

## Example 3

To the aqueous silicone dispersion obtained as in Example 1, was added 111 g (corresponding to 11.1 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 100.8 parts by weight per 100 parts by weight of component (A) and colloidal silica combined.

The aqueous silicone dispersion was dried by the above-mentioned method, obtaining a tacky sheet. The sheet had a Durometer type A rubber hardness of 16, an elongation at break of 230%, and a tensile strength at break of 0.36 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

## Example 4

To the aqueous silicone dispersion obtained as in Example 1, was added 177 g (corresponding to 17.7 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 103.6 parts by weight per 100 parts by weight of component (A) and colloidal silica combined.

The aqueous silicone dispersion was dried by the above-mentioned method, obtaining a non-tacky sheet. The sheet had a Durometer type A rubber hardness of 32, an elongation at break of 270%, and a tensile strength at break of 0.81 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

## Example 5

A glass beaker of volume 1 L was charged with 235 g of dimethylpolysiloxane containing vinyl at both ends of the molecular chain and having a kinematic viscosity of 130 $mm^2/s$ and a vinyl content of 0.035 mol/100 g, as represented by the above formula (A-1'), 25 g of a solid vinyl-containing polysiloxane resin having a vinyl content of 0.086 mol/100 g, represented by the unit formula:

$$[(CH_3)_3SiO_{1/2}]_t[CH_2{=}CH(CH_3)_2SiO_{1/2}]_u[SiO_{4/2}]_v$$

wherein t:u:v = 36:6:58, 119 g of hydrosilyl-containing methylhydrogenpolysiloxane having a kinematic viscosity of 430 $mm^2/s$ and a hydrosilyl content of 0.040 mol/100 g, as represented by the above formula (A-2'), and 121 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3'), which were stirred at 2,000 rpm for dissolution by means of a homo-mixer. The total number of hydrosilyl groups on the hydrosilyl-containing methylhydrogenpolysiloxane of formula (A-2') and the linear dimethylhydrogenpolysiloxane containing hydrosilyl at both ends of the molecular chain of formula (A-3') is 1.22 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3') = 50:50.

Next, 5 g (corresponding to 1.0 part by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 353 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor

shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 8 g of phenoxyethanol as an antibacterial agent was added to the emulsion and agitation was continued at the temperature for 10 minutes, yielding an aqueous silicone dispersion. The content of water in the aqueous silicone dispersion was 96.6 parts by weight per 100 parts by weight of component (A).

On measurement by a laser diffraction/scattering method particle size distribution measuring system LA-960 (Horiba, Ltd.), the silicone elastomer in the aqueous silicone dispersion had a volume average particle size of 780 nm.

The aqueous silicone dispersion was dried by the above-mentioned method, obtaining a tacky sheet. The sheet had an Asker C rubber hardness of 12, an elongation at break of 130%, and a tensile strength at break of 0.14 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

## Example 6

To the aqueous silicone dispersion obtained as in Example 5, was added 177 g (corresponding to 17.7 parts by weight of colloidal silica per 100 parts by weight of a silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 103.6 parts by weight per 100 parts by weight of component (A) and colloidal silica combined.

The aqueous silicone dispersion was dried by the above-mentioned method, obtaining a tacky sheet. The sheet had a Durometer type A rubber hardness of 22, an elongation at break of 540%, and a tensile strength at break of 0.80 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

## Example 7

A glass beaker of volume 1 L was charged with 266 g of dimethylpolysiloxane containing vinyl at both ends of the molecular chain and having a kinematic viscosity of 130 $mm^2/s$ and a vinyl content of 0.035 mol/100 g, as represented by the above formula (A-1'), 162 g of hydrosilyl-containing methylhydrogenpolysiloxane having a kinematic viscosity of 430 $mm^2/s$ and a hydrosilyl content of 0.040 mol/100 g, as represented by the above formula (A-2'), and 72 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3'), which were stirred at 2,000 rpm for dissolution by means of a homo-mixer. The total number of hydrosilyl groups on the hydrosilyl-containing methylhydrogenpolysiloxane of formula (A-2') and the linear dimethylhydrogenpolysiloxane containing hydrosilyl at both ends of the molecular chain of formula (A-3') is 1.21 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3') = 69:31.

Next, 5 g (corresponding to 1.0 part by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 353 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 8 g of phenoxyethanol as an antibacterial agent was added to the emulsion and agitation was continued at the temperature for 10 minutes, yielding an aqueous silicone dispersion.

On measurement by a laser diffraction/scattering method particle size distribution measuring system LA-960 (Horiba, Ltd.), the silicone elastomer in the aqueous silicone dispersion had a volume average particle size of 850 nm.

To the aqueous silicone dispersion was added 53 g (corresponding to 5.3 parts by weight of colloidal silica per 100 parts by weight of a silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 96.9 parts by weight per 100 parts by weight of component (A) and colloidal silica combined.

The aqueous silicone dispersion was dried by the above-mentioned method, obtaining a non-tacky sheet. The sheet had a Durometer type A hardness of 13, an elongation at break of 25%, and a tensile strength at break of 0.11 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

Example 8

A glass beaker of volume 1 L was charged with 275 g of dimethylpolysiloxane containing vinyl at both ends of the molecular chain and having a kinematic viscosity of 130 $mm^2/s$ and a vinyl content of 0.035 mol/100 g, as represented by the above formula (A-1'), 112 g of hydrosilyl-containing methylhydrogenpolysiloxane having a kinematic viscosity of 430 $mm^2/s$ and a hydrosilyl content of 0.040 mol/100 g, as represented by the above formula (A-2'), and 113 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3'), which were stirred at 2,000 rpm for dissolution by means of a homo-mixer. The total number of hydrosilyl groups on the hydrosilyl-containing methylhydrogenpolysiloxane of formula (A-2') and the linear dimethylhydrogenpolysiloxane containing hydrosilyl at both ends of the molecular chain of formula (A-3') is 1.24 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3') = 50:50.

Next, 5 g (corresponding to 1.0 part by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 353 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 8 g of phenoxyethanol as an antibacterial agent was added to the emulsion and agitation was continued at the temperature for 10 minutes, yielding an aqueous silicone dispersion.

On measurement by a laser diffraction/scattering method particle size distribution measuring system LA-960 (Horiba, Ltd.), the silicone elastomer in the aqueous silicone dispersion had a volume average particle size of 830 nm.

To the aqueous silicone dispersion was added 177 g (corresponding to 17.7 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 103.6 parts by weight per 100 parts by weight of component (A) and colloidal silica combined.

The aqueous silicone dispersion was dried by the above-mentioned method, obtaining a non-tacky sheet. The sheet had a Durometer type A hardness of 32, an elongation at break of 270%, and a tensile strength at break of 0.81 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

Example 9

A glass beaker of volume 1 L was charged with 325 g of dimethylpolysiloxane containing vinyl at both ends of the molecular chain and having a kinematic viscosity of 130 $mm^2/s$ and a vinyl content of 0.035 mol/100 g, as represented

by the above formula (A-1’), 172 g of hydrosilyl-containing methylhydrogenpolysiloxane having a kinematic viscosity of 430 $mm^2/s$ and a hydrosilyl content of 0.040 mol/100 g, as represented by the above formula (A-2’), and 103 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3’), which were stirred at 2,000 rpm for dissolution by means of a homo-mixer. The total number of hydrosilyl groups on the hydrosilyl-containing methylhydrogenpolysiloxane of formula (A-2’) and the linear dimethylhydrogenpolysiloxane containing hydrosilyl at both ends of the molecular chain of formula (A-3’) is 1.20 per vinyl group on the vinyl-containing dimethylpolysiloxane of formula (A-1’). A weight ratio of the hydrosilyl-containing methylhydrogenpolysiloxane of formula (A-2’) to the linear dimethylhydrogenpolysiloxane containing hydrosilyl at both ends of the molecular chain of formula (A-3’) is (A-2’):(A-3’) = 63:37.

Next, 6 g (corresponding to 1.0 part by weight per 100 parts by weight of a silicone elastomer) of sodium laurylsulfate, 10 g of phenoxyethanol as an antibacterial agent, and 160 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 222 g of water was added to the emulsion for dilution. It was passed through a homogenizer under a pressure of 100 MPa, obtaining a uniform white emulsion.

The emulsion, 798 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 0.6 g (corresponding to 0.13 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1’) with the hydrosilyl-containing methylhydrogenpolysiloxane of formula (A-2’) and the linear dimethylhydrogenpolysiloxane containing hydrosilyl at both ends of the molecular chain of formula (A-3’). An aqueous silicone dispersion was obtained.

On measurement by a laser diffraction/scattering method particle size distribution measuring system LA-960 (Horiba, Ltd.), the silicone elastomer in the aqueous silicone dispersion had a volume average particle size of 630 nm.

To the aqueous silicone dispersion was added 212 g (corresponding to 17.7 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 76.6 parts by weight per 100 parts by weight of component (A) and colloidal silica combined.

The aqueous silicone dispersion was dried by the above-mentioned method, obtaining a non-tacky sheet. The sheet had a Durometer type A hardness of 42, an elongation at break of 200%, and a tensile strength at break of 1.1 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

Example 10

A glass beaker of volume 1 L was charged with 275 g of dimethylpolysiloxane containing vinyl at both ends of the molecular chain and having a kinematic viscosity of 130 $mm^2/s$ and a vinyl content of 0.035 mol/100 g, as represented by the above formula (A-1’), 112 g of hydrosilyl-containing methylhydrogenpolysiloxane having a kinematic viscosity of 430 $mm^2/s$ and a hydrosilyl content of 0.040 mol/100 g, as represented by the above formula (A-2’), and 113 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3’), which were stirred at 2,000 rpm for dissolution by means of a homo-mixer. The total number of hydrosilyl groups on the hydrosilyl-containing methylhydrogenpolysiloxane of formula (A-2’) and the linear dimethylhydrogenpolysiloxane containing hydrosilyl at both ends of the molecular chain of formula (A-3’) is 1.24 per vinyl group on the vinyl-containing dimethylpolysiloxane of formula (A-1’). A weight ratio of the hydrosilyl-containing methylhydrogenpolysiloxane of formula (A-2’) to the linear dimethylhydrogenpolysiloxane containing hydrosilyl at both ends of the molecular chain of formula (A-3’) is (A-2’):(A-3’) = 50:50.

Next, 10 g (corresponding to 2.0 parts by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 348 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane

solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 8 g of phenoxyethanol as an antibacterial agent was added to the emulsion and agitation was continued at the temperature for 10 minutes, yielding an aqueous silicone dispersion.

On measurement by a laser diffraction/scattering method particle size distribution measuring system LA-960 (Horiba, Ltd.), the silicone elastomer in the aqueous silicone dispersion had a volume average particle size of 670 nm.

To the aqueous silicone dispersion was added 177 g (corresponding to 17.7 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 103.8 parts by weight per 100 parts by weight of component (A) and colloidal silica combined.

The aqueous silicone dispersion was dried by the above-mentioned method, obtaining a non-tacky sheet. The sheet had a Durometer type A hardness of 32, an elongation at break of 120%, and a tensile strength at break of 0.67 MPa.

A about 0.02 g portion of the aqueous silicone dispersion was dropped from a pipette on the back of the hand and spread over a diameter of about 2 cm with the finger. After air drying for 3 minutes, the spread was intensely rubbed with the finger. The spread was twisted, and solid strings dropped. It was judged that a soft film had been formed.

Comparative Example 1

A glass beaker of volume 1 L was charged with 244 g of dimethylpolysiloxane containing vinyl at both ends of the molecular chain and having a kinematic viscosity of 130 $mm^2/s$ and a vinyl content of 0.035 mol/100 g, as represented by the above formula (A-1') and 256 g of hydrosilyl-containing methylhydrogenpolysiloxane having a kinematic viscosity of 430 $mm^2/s$ and a hydrosilyl content of 0.040 mol/100 g, as represented by the above formula (A-2'), which were stirred at 2,000 rpm for dissolution by means of a homo-mixer. The number of hydrosilyl groups on the hydrosilyl-containing methylhydrogenpolysiloxane of formula (A-2') is 1.20 per vinyl group on the vinyl-containing dimethylpolysiloxane of formula (A-1'). This example is a composition not containing a linear dimethylhydrogenpolysiloxane containing the hydrosilyl at the ends of the molecular chain, among the hydrosilyl-containing methylpolysiloxanes.

Next, 5 g (corresponding to 1.0 part by weight per 100 parts by weight of a silicone elastomer) of sodium laurylsulfate and 130 g of water were added to the solution, which was agitated at 8,000 rpm by a homo-mixer. There was formed an oil-in-water type emulsion with a viscosity buildup observed, and agitation was continued for a further 15 minutes. With stirring at 2,000 rpm, 353 g of water was added to the emulsion for dilution. It was passed through a homogenizer under a pressure of 100 MPa, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2'). Then 10 g of phenoxyethanol as an antibacterial agent was added to the emulsion and agitation was continued at the temperature for 10 minutes, yielding an aqueous silicone dispersion. The content of water in the aqueous silicone dispersion was 96.6 parts by weight per 100 parts by weight of component (A).

On measurement by a laser diffraction/scattering method particle size distribution measuring system LA-960 (Horiba, Ltd.), the silicone elastomer in the aqueous silicone dispersion had a volume average particle size of 850 nm.

The aqueous silicone dispersion was dried by the above-mentioned method, obtaining a non-tacky dry product. The product was too brittle to peel it in sheet form from the tray.

Comparative Example 2

A glass beaker of volume 1 L was charged with 271 g of dimethylpolysiloxane containing vinyl at both ends of the molecular chain and having a kinematic viscosity of 130 $mm^2/s$ and a vinyl content of 0.035 mol/100 g, as represented by the above formula (A-1'), 137 g of hydrosilyl-containing methylhydrogenpolysiloxane having a kinematic viscosity of 430 $mm^2/s$ and a hydrosilyl content of 0.040 mol/100 g, as represented by the above formula (A-2'), and 92 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3'), which were stirred at 2,000 rpm for dissolution by means of a homo-mixer. The total number of hydrosilyl groups on the hydrosilyl-containing

methylhydrogenpolysiloxane of formula (A-2') and the linear dimethylhydrogenpolysiloxane containing hydrosilyl at both ends of the molecular chain of formula (A-3') is 1.22 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3') = 60:40.

Next, 45 g (corresponding to 9.0 parts by weight per 100 parts by weight of a silicone elastomer) of sodium laurylsulfate and 60 g of water were added to the solution, which was agitated at 4,000 rpm by a homo-mixer. The emulsion became of oil-in-water type and greasy, and agitation was continued for a further 10 minutes. With stirring at 2,000 rpm by a homo-mixer, 383 g of water was added to the emulsion, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 10 g of phenoxyethanol as an antibacterial agent was added to the emulsion and agitation was continued at the temperature for 10 minutes, yielding an aqueous silicone dispersion. The water content in the aqueous silicone dispersion was 88.6 parts by weight per 100 parts by weight of component (A).

On measurement by a laser diffraction/scattering method particle size distribution measuring system LA-960 (Horiba, Ltd.), the silicone elastomer in the aqueous silicone dispersion had a volume average particle size of 470 nm.

The aqueous silicone dispersion was dried by the above-mentioned method, obtaining a non-tacky dry product. The product was too brittle to peel it in sheet form from the tray.

<u>Comparative Example 3</u>

To the aqueous silicone dispersion obtained as in Comparative Example 1, was added 53 g (corresponding to 5.3 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 91.7 parts by weight per 100 parts by weight of component (A) and colloidal silica combined.

The aqueous silicone dispersion was dried by the above-mentioned method, obtaining a non-tacky dry product. The product was too brittle to peel it in sheet form from the tray.

<u>Comparative Example 4</u>

An aqueous silicone dispersion was prepared by the same procedure as in Example 1 aside from using 5 g of polyoxyethylene lauryl ether (moles of ethylene oxide added = 9 mol) instead of 5 g of sodium laurylsulfate used in Example 1. The content of water in the aqueous silicone dispersion was 96.6 parts by weight per 100 parts by weight of component (A).

On measurement by a laser diffraction/scattering method particle size distribution measuring system LA-960 (Horiba, Ltd.), the silicone elastomer in the aqueous silicone dispersion had a volume average particle size of 720 nm.

The aqueous silicone dispersion was dried by the above-mentioned method, obtaining a non-tacky dry product. The product was too brittle to peel it in sheet form from the tray.

<u>Comparative Example 5</u>

A glass beaker of volume 1 L was charged with 271 g of dimethylpolysiloxane containing vinyl at both ends of the molecular chain and having a kinematic viscosity of 130 $mm^2/s$ and a vinyl content of 0.035 mol/100 g, as represented by the above formula (A-1'), 137 g of hydrosilyl-containing methylhydrogenpolysiloxane having a kinematic viscosity of 430 $mm^2/s$ and a hydrosilyl content of 0.040 mol/100 g, as represented by the above formula (A-2'), and 92 g of linear dimethylhydrogenpolysiloxane containing hydrosilyl at both ends of the molecular chain and having a kinematic viscosity of 35 $mm^2/s$ and a hydrosilyl content of 0.066 mol/100 g, as represented by the above formula (A-3'), which were stirred at 2,000 rpm for dissolution by means of a homo-mixer. The total number of hydrosilyl groups on the hydrosilyl-containing methylhydrogenpolysiloxane of formula (A-2') and the linear dimethylhydrogenpolysiloxane containing hydrosilyl at both ends of the molecular chain of formula (A-3') is 1.22 per vinyl group on the vinyl-containing dimethylpolysiloxane of formula (A-1'). A weight ratio of the hydrosilyl-containing methylhydrogenpolysiloxane of formula (A-2') to the linear dimethylhydrogenpolysiloxane containing hydrosilyl at both ends of the molecular chain of formula (A-3') is (A-2'):(A-3')

= 60:40.

Next, 5 g (corresponding to 1.0 parts by weight per 100 parts by weight of a silicone elastomer) of sodium laurylsulfate, 25 g of polyoxyethylene lauryl ether (moles of ethylene oxide added = 9 mol), and 40 g of water were added to the solution, which was agitated at 4,000 rpm by a homo-disper. The emulsion became of oil-in-water type and greasy, and agitation was continued for a further 10 minutes. With stirring at 2,000 rpm by a homo-mixer, 418 g of water was added to the emulsion, obtaining a uniform white emulsion.

The emulsion, 790 g, was transferred to a glass flask of volume 2 L equipped with an agitator having an anchor shape impeller. After temperature conditioning at 20-25°C, with stirring, a dissolved mixture of 0.6 g of an isododecane solution of platinum/vinyl-containing siloxane complex (Pt content 0.5%) and 1.1 g (corresponding to 0.28 part by weight per 100 parts by weight of a silicone elastomer) of polyoxyethylene lauryl ether (moles of ethylene oxide added = 9 mol) was added to the emulsion. The contents were agitated at the temperature for 12 hours, for thereby effecting addition reaction of the vinyl-containing dimethylpolysiloxane of formula (A-1') with the hydrosilyl-containing methylhydrogenpolysiloxane of formula (A-2') and the linear dimethylhydrogenpolysiloxane containing hydrosilyl at both ends of the molecular chain of formula (A-3'). Then 10 g of phenoxyethanol as an antibacterial agent was added to the emulsion and agitation was continued at the temperature for 10 minutes, yielding an aqueous silicone dispersion. The water content in the aqueous silicone dispersion was 91.6 parts by weight per 100 parts by weight of component (A).

On measurement by a laser diffraction/scattering method particle size distribution measuring system LA-960 (Horiba, Ltd.), the silicone elastomer in the aqueous silicone dispersion had a volume average particle size of 660 nm.

The aqueous silicone dispersion was dried by the above-mentioned method, obtaining a non-tacky dry product. The product was too brittle to peel it in sheet form from the tray.

Comparative Example 6

To the aqueous silicone dispersion obtained as in Example 1, was added 360 g (corresponding to 36.0 parts by weight of colloidal silica per 100 parts by weight of the silicone elastomer) of a colloidal silica water dispersion having a concentration of 40% (trade name COSMO S-40, by JGC C&C). This was agitated for 10 minutes, yielding an aqueous silicone dispersion. The content of water in the aqueous silicone dispersion was 109.8 parts by weight per 100 parts by weight of component (A) and colloidal silica combined.

The aqueous silicone dispersion was dried by the above-mentioned method, obtaining a non-tacky dry product. The product was too brittle to peel it in sheet form from the tray.

Comparative Example 7

A glass beaker of volume 1 L was charged with 500 g of octamethylcyclotetrasiloxane, 30 g of dodecylbenzenesulfonic acid as an emulsifier/polymerization catalyst, and 170 g of water, which were stirred at 8,000 rpm by a homomixer. There was formed an oil-in-water type emulsion with a viscosity buildup observed, and agitation was continued for a further 15 minutes. With stirring at 2,000 rpm, 300 g of water was added to the emulsion for dilution. It was passed through a homogenizer under a pressure of 30 MPa, obtaining a uniform white emulsion.

The emulsion, 800 g, was transferred to a glass flask of volume 2 L equipped with an agitator having an anchor shape impeller, followed by reaction at 70°C for 6 hours and aging at 15°C for 12 hours. Thereafter, 44 g of 10 wt% sodium carbonate aqueous solution was added to the emulsion to neutralize at pH 6.2. The polysiloxane value in the resulting emulsion was a dimethylpolysiloxane containing hydroxyl groups at both ends of the molecular chain. Isopropyl alcohol was added to the emulsion to break the emulsion to separate the polysiloxane. On analysis, the polysiloxane had a complex viscosity of $2.3 \times 10^6$ mPa·s.

With stirring, 8 g of phenyltriethoxysilane was added to 787 g of the resulting emulsion of the dimethylpolysiloxane containing hydroxyl groups at both ends of the molecular chain, which was stirred for 1 hour. Further, 4.8 g of an emulsion of dioctyltin diversatate as a condensation catalyst (dioctyltin diversatate concentration 42%) and 8 g of phenoxyethanol as an antibacterial agent were added to the emulsion, which was stirred for 10 minutes, yielding an aqueous silicone dispersion. The content of water in the aqueous silicone dispersion was 103.2 parts by weight per 100 parts by weight of the polysiloxane and phenyltriethoxysilane combined.

On measurement by a laser diffraction/scattering method particle size distribution measuring system LA-960 (Horiba, Ltd.), the silicone elastomer in the aqueous silicone dispersion had a volume average particle size of 220 nm.

The aqueous silicone dispersion was dried by the above-mentioned method, obtaining a non-tacky sheet. The sheet had a Durometer type A hardness of 12, an elongation at break of 860%, and a tensile strength at break of 0.60 MPa.

A portion of about 0.02 g of the aqueous silicone dispersion was dropped from a pipette onto the back of the hand and spread over a diameter of about 2 cm with the finger. After air drying for 3 minutes, the spread was intensely rubbed with the finger, but no solids dropped. On brief drying, a cured film did not form.

## Claims

1. An aqueous silicone dispersion of silicone elastomer, which forms an elastomer film upon drying at normal temperature, the dispersion comprising:

   (A) the dispersed silicone elastomer, which is the addition reaction product of

      (A-1) alkenyl-containing organopolysiloxane having at least 2 alkenyl groups per molecule with
      (A-2) organohydrogenpolysiloxane having at least 3 hydrosilyl groups per molecule, and
      (A-3) linear diorganohydrogenpolysiloxane having hydrosilyl groups at both ends of the molecular chain,

   (B) anionic surfactant in an amount of 0.1 to 5 parts by weight per 100 parts by weight of component (A),
   (C) nonionic surfactant in an amount of 0 to 2 parts by weight per 100 parts by weight of component (A),
   (D) colloidal silica in an amount of 0 to 35 parts by weight per 100 parts by weight of component (A), and
   (E) water in an amount of 15 to 200 parts by weight per 100 parts by weight of components (A) and (D) combined.

2. Aqueous silicone dispersion of claim 1 wherein an elastomer sheet of 1 mm thickness obtained by drying the aqueous silicone dispersion at 25°C has Asker C rubber hardness of at least 5 as measured by the testing method of the Society of Rubber Industry, Japan Standard (SRIS).

3. Aqueous silicone dispersion of claim 1 or 2 wherein an elastomer sheet of 1 mm thickness obtained by drying the aqueous silicone dispersion at 25°C has an elongation at break of at least 20% and a tensile strength at break of at least 0.05 MPa when a dumbbell shaped #3 specimen is measured by the testing method of JIS K6251.

4. Aqueous silicone dispersion of any one of claims 1 to 3 wherein component (A) is the addition reaction product of component (A-1) with components (A-2) and (A-3), the weight ratio of component (A-2) to component (A-3) being in the range from 5:95 to 90:10.

5. Aqueous silicone dispersion of any one of claims 1 to 4 wherein component (A-1) has the average compositional formula (1):

   $$R^1_aR^2_bSiO_{(4-a-b)/2} \qquad (1)$$

   wherein $R^1$ is independently a $C_1$-$C_{30}$ substituted or unsubstituted monovalent hydrocarbon group exclusive of alkenyl, $R^2$ is independently a $C_2$-$C_6$ alkenyl group, a and b are positive numbers satisfying $0 < a < 3$, $0 < b \leq 3$, and $0.1 \leq a+b \leq 3$.

6. Aqueous silicone dispersion of any one of claims 1 to 5 wherein component (A-2) has the average compositional formula (6):

   $$R^3_wH_xSiO_{(4-w-x)/2} \qquad (6)$$

   wherein $R^3$ is independently a $C_1$-$C_{30}$ substituted or unsubstituted monovalent hydrocarbon group exclusive of alkenyl, w and x are positive numbers satisfying $0 < w < 3$, $0 < x \leq 3$, and $0.1 \leq w+x \leq 3$.

7. Aqueous silicone dispersion of any one of claims 1 to 6 wherein component (A-3) has 2 hydrosilyl groups per molecule.

8. Aqueous silicone dispersion of claim 7 wherein component (A-3) is represented by the general formula (11):

   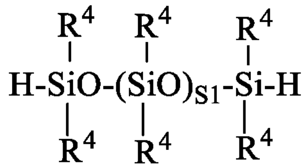

   (11)

   wherein $R^4$ is independently a $C_1$-$C_{30}$ substituted or unsubstituted monovalent hydrocarbon group exclusive of alkenyl, and s1 is a positive number of 5 to 1,000.

9. Aqueous silicone dispersion of any one of claims 1 to 8 wherein the dispersed silicone elastomer (A) has a volume average particle size of up to 10 μm, measured by the laser diffraction/scattering type particle size measuring method.

10. An elastomer film obtained by drying an aqueous silicone dispersion of any one of claims 1 to 9 at normal temperature.

11. A cosmetic composition comprising an aqueous silicone dispersion of any one of claims 1 to 9.

12. A cosmetic composition of claim 11 which is selected from make-up cosmetics, pack cosmetics and eyelash cosmetics.

13. A method of preparing an aqueous silicone dispersion of any one of claims 1 to 9, comprising the steps of:

emulsifying an alkenyl-containing organopolysiloxane (A-1) and hydrosilyl-containing organohydrogenpolysiloxanes (A-2) and (A-3) as reactants for the silicone elastomer (A) in water as component (E) with the aid of an anionic surfactant as component (B),
adding a platinum group metal base catalyst to the emulsion, and
effecting addition reaction,

wherein optionally a nonionic surfactant as component (C) is added in the emulsifying step, and optionally colloidal silica as component (D) is added after the emulsifying step or after the addition reaction step.

**Patentansprüche**

1. Wässrige Silikon-Dispersion eines Silikon-Elastomers, die nach dem Trocknen bei Normaltemperatur einen Elastomer-Film bildet, wobei die Dispersion Folgendes umfasst:

(A) das dispergierte Silikon-Elastomer, welches das Additionsreaktionsprodukt von

(A-1) Alkenyl-hältigem Organopolysiloxan mit zumindest 2 Alkenylgruppen pro Molekül mit
(A-2) Organohydrogenpolysiloxan mit zumindest 3 Hydrosilylgruppen pro Molekül und
(A-3) unverzweigtem Diorganohydrogenpolysiloxan mit Hydrosilylgruppen an beiden Enden der Molekülkette ist,

(B) anionisches Tensid in einer Menge von 0,1 bis 5 Gewichtsteilen, bezogen auf 100 Gewichtsteile der Komponente (A),
(C) nichtionisches Tensid in einer Menge von 0 bis 2 Gewichtsteile, bezogen auf 100 Gewichtsteile der Komponente (A),
(D) kolloidales Siliciumdioxid in einer Menge von 0 bis 35 Gewichtsteilen, bezogen auf 100 Gewichtsteile der Komponente (A) und
(E) Wasser in einer Menge von 15 bis 200 Gewichtsteilen, bezogen auf 100 Gewichtsteile der Komponenten (A) und (D) zusammen.

2. Wässrige Silikondispersion nach Anspruch 1, wobei eine Elastomer-Lage mit 1 mm Dicke, die durch Trocknen der wässrigen Silikondispersion bei 25 °C erhalten wird, eine Asker-C-Kautschukhärte von zumindest 5 aufweist, wie nach dem Prüfverfahren der Society of Rubber Industry, Japan Standard (SRIS), gemessen.

3. Wässrige Silikondispersion nach Anspruch 1 oder 2, wobei eine Elastomer-Lage mit 1 mm Dicke, die durch Trocknen der wässrigen Silikondispersion bei 25 °C erhalten wird, eine Reißdehnung von zumindest 20 % und eine Reißfestigkeit von zumindest 0,05 MPa aufweist, wenn ein Prüfling in Form einer Hantel Nr. 3 nach dem Prüfverfahren von JIS K6251 gemessen wird.

4. Wässrige Silikondispersion nach einem der Ansprüche 1 bis 3, wobei Komponente (A) das Additionsreaktionsprodukt von Komponente (A-1) mit Komponente (A-2) und (A-3) ist, wobei das Gewichtsverhältnis zwischen Komponente (A-2) und Komponente (A-3) im Bereich von 5:95 bis 90:10 liegt.

5. Wässrige Silikondispersion nach einem der Ansprüche 1 bis 4, wobei Komponente (A-1) die folgende durchschnittliche Zusammensetzungsformel (1) aufweist:

$R^1_4R^2_bSiO_{(4-a-b)/2}$ (1)

worin $R^1$ unabhängig eine substituierte oder unsubstituierte einwertige $C_1$-$C_{30}$-Kohlenwasserstoffgruppe außer Alkenyl ist, $R^2$ unabhängig eine $C_2$-$C_6$-Alkenylgruppe ist und a und b positive Zahlen sind, die $0 < a < 3$, $0 < b \leq 3$ und $0{,}1 \leq a+b \leq 3$ erfüllen.

**6.** Wässrige Silikondispersion nach einem der Ansprüche 1 bis 5, wobei Komponente (A-2) die folgende durchschnittliche Zusammensetzungsformel (6) aufweist:

$R^3_wH_xSiO_{(4-w-x)/2}$ (6)

worin $R^3$ unabhängig eine substituierte oder unsubstituierte einwertige $C_1$-$C_{30}$-Kohlenwasserstoffgruppe außer Alkenyl ist und w und x positive Zahlen sind, die $0 < w < 3$, $0 < x \leq 3$ und $0{,}1 \leq w+x \leq 3$ erfüllen.

**7.** Wässrige Silikondispersion nach einem der Ansprüche 1 bis 6, wobei Komponente (A-3) 2 Hydrosilylgruppen pro Molekül aufweist.

**8.** Wässrige Silikondispersion nach Anspruch 7, wobei Komponente (A-3) durch die folgende allgemeine Formel (11) dargestellt ist:

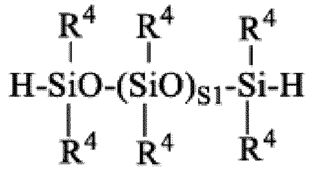

(11)

worin $R^4$ unabhängig eine substituierte oder unsubstituierte einwertige $C_1$-$C_{30}$-Kohlenwasserstoffgruppe außer Alkenyl ist und s1 eine positive Zahl von 5 bis 1.000 ist.

**9.** Wässrige Silikondispersion nach einem der Ansprüche 1 bis 8, wobei das dispergierte Silikon-Elastomer (A) eine volumenmittlere Teilchengröße von bis zu 10 μm aufweist, gemessen durch das Teilchengrößenmessverfahren vom Laserbeugungs-/-streuungs-Typ.

**10.** Elastomerfolie, die durch Trocknen einer wässrigen Silikondispersion nach einem der Ansprüche 1 bis 9 bei Normaltemperatur erhältlich ist.

**11.** Kosmetikzusammensetzung, die eine wässrige Silikondispersion nach einem der Ansprüche 1 bis 9 umfasst.

**12.** Kosmetikzusammensetzung nach Anspruch 11, die aus Make-up-Kosmetik, Masken-Kosmetik und Wimpern-Kosmetik ausgewählt ist.

**13.** Verfahren zur Herstellung einer wässrigen Silikondispersion nach einem der Ansprüche 1 bis 9, das die folgenden Schritte umfasst:

das Emulgieren eines Alkenyl-hältigen Organopolysiloxans (A-1) und von Hydrosilylhältigen Organohydrogenpolysiloxanen (A-2) und (A-3) als Reaktanten für das Silikon-Elastomer (A) in Wasser als Komponente (E) mithilfe eines anionischen Tensids als Komponente (B),
das Zusetzen eines Platingruppenmetall-Basenkatalysators zu der Emulsion und
das Durchführen einer Additionsreaktion,

wobei gegebenenfalls ein nichtionisches Tensid als Komponente (C) im Emulgierungsschritt zugesetzt wird und wobei gegebenenfalls kolloidales Siliciumdioxid als Komponente (D) nach dem Emulgierungsschritt oder nach dem Additionsreaktionsschritt zugesetzt wird.

## Revendications

1. Dispersion de silicone aqueuse d'élastomère de silicone, qui forme un film d'élastomère lors d'un séchage à température ambiante, la dispersion comprenant :

   (A) l'élastomère de silicone dispersé, qui est le produit de réaction d'addition d'un

   (A-1) organopolysiloxane contenant un alcényle présentant au moins 2 groupes alcényle par molécule avec un
   (A-2) organohydrogénopolysiloxane présentant au moins 3 groupes hydrosilyle par molécule, et
   (A-3) un diorganohydrogénopolysiloxane linéaire présentant des groupes hydrosilyle aux deux extrémités de la chaîne moléculaire,

   (B) un tensioactif anionique en une quantité de 0,1 à 5 parties en poids pour 100 parties en poids du composant (A),
   (C) un tensioactif anionique en une quantité de 0 à 2 parties en poids pour 100 parties en poids du composant (A),
   (D) de la silice colloïdale en une quantité de 0 à 35 parties en poids pour 100 parties en poids du composant (A), et
   (E) de l'eau en une quantité de 15 à 200 parties en poids pour 100 parties en poids des composants (A), et (D) combinés.

2. Dispersion de silicone aqueuse selon la revendication 1, dans laquelle une feuille d'élastomère de 1 mm d'épaisseur obtenue par séchage de la dispersion de silicone aqueuse à 25°C présente une dureté de caoutchouc Asker C d'au moins 5 telle que mesurée par la méthode de test de la Society of Rubber Industry, Japan Standard (SRIS).

3. Dispersion de silicone aqueuse selon la revendication 1 ou 2, dans laquelle une feuille d'élastomère de 1 mm d'épaisseur obtenue par séchage de la dispersion de silicone aqueuse à 25°C présente un allongement à la rupture d'au moins 20 % et une résistance à la traction à la rupture d'au moins 0,05 MPa lorsqu'un échantillon en forme d'haltère #3 est mesuré par la méthode de test de JIS K6251.

4. Dispersion de silicone aqueuse selon l'une quelconque des revendications 1 à 3, dans laquelle le composant (A) est le produit de réaction d'addition du composant (A-1) avec les composants (A-2) et (A-3), le rapport pondéral du composant (A-2) au composant (A-3) étant dans la plage de 5:95 à 90:10.

5. Dispersion de silicone aqueuse selon l'une quelconque des revendications 1 à 4, dans laquelle le composant (A-1) présente la formule compositionnelle moyenne (1) :

   $$R^1_aR^2_bSiO_{(4-a-b)/2} \qquad (1)$$

   dans laquelle $R^1$ est indépendamment un groupe hydrocarboné monovalent substitué ou non substitué en $C_1$-$C_{30}$ à l'exclusion de l'alcényle, $R^2$ est indépendamment un groupe alcényle en $C_2$-$C_6$, a et b sont des nombres positifs satisfaisant $0 < a < 3$, $0 < b \leq 3$, et $0,1 \leq a+b \leq 3$.

6. Dispersion de silicone aqueuse selon l'une quelconque des revendications 1 à 5, dans laquelle le composant (A-2) présente la formule compositionnelle moyenne (6) :

   $$R^3_wH_xSiO_{(4-w-x)/2} \qquad (6)$$

   dans laquelle $R^3$ est indépendamment un groupe hydrocarboné monovalent substitué ou non substitué en $C_1$-$C_{30}$ à l'exclusion de l'alcényle, w et x sont des nombres positifs satisfaisant $0 < w < 3$, $0 < x \leq 3$, et $0,1 \leq w+x \leq 3$.

7. Dispersion de silicone aqueuse selon l'une quelconque des revendications 1 à 6, dans laquelle le composant (A-3) présente 2 groupes hydrosilyle par molécule.

8. Dispersion de silicone aqueuse selon la revendication 7, dans laquelle le composant (A-3) est représenté par la formule générale (11) :

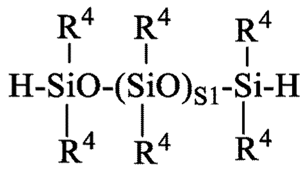

(11)

dans laquelle $R^4$ est indépendamment un groupe hydrocarboné monovalent substitué ou non substitué en $C_1$-$C_{30}$ à l'exclusion de l'alcényle, et s1 est un nombre positif compris entre 5 et 1 000.

**9.** Dispersion de silicone aqueuse selon l'une quelconque des revendications 1 à 8, dans laquelle l'élastomère de silicone dispersé (A) présente une taille de particule moyenne en volume allant jusqu'à 10 um, mesurée par le procédé de mesure de taille de particule de type par diffraction/diffusion laser.

**10.** Film d'élastomère obtenu par séchage d'une dispersion de silicone aqueuse selon l'une quelconque des revendications 1 à 9 à température normale.

**11.** Composition cosmétique comprenant une dispersion de silicone aqueuse selon l'une quelconque des revendications 1 à 9.

**12.** Composition cosmétique selon la revendication 11, qui est choisie parmi des cosmétiques de maquillage, des cosmétiques de masque et les cosmétiques pour les cils.

**13.** Procédé de préparation d'une composition de silicone selon l'une quelconque des revendications 1 à 9, comprenant les étapes consistant à :

émulsionner un organopolysiloxane contenant un alcényle (A-1) et des organohydrogénopolysiloxanes contenant un hydrosilyle (A-2) et (A-3) en tant que réactifs pour l'élastomère de silicone (A) dans de l'eau en tant que composant (E) à l'aide d'un tensioactif anionique en tant que composant (B),
ajouter un catalyseur à base de métal du groupe platine à l'émulsion, et
effectuer une réaction d'addition,
dans lequel facultativement un tensioactif non ionique en tant que composant (C) est ajouté dans l'étape d'émulsion, et facultativement de la silice colloïdale en tant que composant (D) est ajoutée après l'étape d'émulsion ou après l'étape de réaction d'addition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3098833 A
- JP S38860 B
- JP S53130752 A
- US 3294725 A
- JP S54131661 A
- JP H07196984 A
- JP S5094082 A
- JP S5452160 A
- JP S5636546 A
- WO 2016164296 A
- EP 1361253 A1
- WO 2008057155 A1
- EP 1536762 A2
- EP 2357024 A2
- US 3220972 A
- US 3159601 A
- US 3159662 A
- US 3775452 A